# EUROPEAN PATENT APPLICATION

(11) **EP 0 850 612 A1**
(43) Date of publication of application: **01.07.1998**
(21) Application number: 97929546.6
(22) Date of filing: 07.07.1997
(51) Int. Cl.: A61F 5/441, A61F 5/445

(54) **EXCREMENT COLLECTING BAG**

(30) Priority: 05.07.1996 JP 195612/96
(71) Applicant: Alcare Co., Ltd., Sumida-ku, Tokyo 131 (JP)
(72) Inventor: TAKAHASHI, Tetsuya, Haimu Matsuno 201, Tokyo 141 (JP); SANADA, Hiromi, Kanazawa-shi, Ishikawa 920-01 (JP); KONYA, Chizuko, Kanazawa-shi, Ishikawa 920 (JP)
(74) Representative: Preissner, Nicolaus, Dipl.-Ing.
(86) International application number: JP9702347
(87) International publication number: WO9801093

(57) **Abstract**

The body wastes receiving pouch appliance according to the present invention is detachably secured to a body wastes discharge hole or opening of a human body; said pouch appliance comprises a pouch member 5 for receiving and storing body wastes 17, and a deodorant pouring inlet port 7 and a non-return valve 9 are provided in said pouch member, so that, when the body wastes are to be removed, a deodorant is poured into the pouch member through the deodorant pouring inlet port, whereby the production of an odor is prevented.

## Description

### [Technical Field]

The present invention relates to a body wastes receiving pouch appliance for receiving the body wastes discharged from a body wastes discharge hole or opening of a human body.

### [Background Technique]

The urine and feces discharged by incontinence from the natural excretory holes or openings, that is, the urethral meatus and the anus, of a human body, the excrements discharged from an excretory hole or opening such as, e.g. an artificial anus or an artificial urinary bladder which has been formed by artificially altering the natural excretory hole or opening due to a disease, or the various body wastes discharged from a fistulous hole or opening which has resulted from a disease or a fistulous hole or opening artificially formed for medical treatment cannot be controlled by the human body's own natural functions; and therefore, ordinarily a body wastes receiving pouch appliance is secured to or around the body wastes discharge opening or fistulous opening (hereinafter referred to simply as the body wastes discharge opening) to receive the body wastes naturally discharged therefrom, so that the patient can pass his daily life smoothly.

This body wastes receiving pouch appliance is kept in a state hermetically sealed from the outside during use, so that, if only the pouch member thereof is made to contain a deodorant (such as, e.g. polyvinylidene chloride) as a material thereof, the odor of the body wastes received is not allowed to leak out even if the body wastes receiving pouch appliance is used in the state in which the thus received body wastes are stored in said pouch member, but, as soon as the pouch member is removed from the body wastes from within the pouch member, the odor leaks out from the opening portion of said pouch member, thus causing the people around as well as the patient himself feel bad. In order to avoid this, it may be considered to use a deodorant when the body wastes are taken out for removal from the pouch member, but it is not possible to prevent the odor from leaking out when the pouch member is released. Further, it is considered to use the method of previously incorporating a deodorant in the receiving pouch member, but, in this case, the deodorizing action of said deodorant starts to take effect from the point of time when the deodorant is put into the receiving pouch member regardless of whether any body wastes are present in the receiving pouch member or not; and thus, this method is very uneconomical. Further, the duration of the deodorizing effect of said deodorant has its limit, and it is very unsanitary and troublesome to remove only the body wastes, leaving the still effective deodorant inside the receiving pouch member or to insert a new deodorant into the receiving pouch member after the removal of only the body wastes from within said receiving pouch member which is still kept in the state secured to the human body.

### [Disclosure of the Invention]

It is the object of the present invention to prevent an odor from being produced when the body wastes are removed from a body wastes receiving pouch appliance.

In order to give a solution to the above-mentioned prom according to the present invention, a deodorant pouring inlet port and a non-return valve are provided in a body wastes receiving pouch appliance comprising a pouch member in which the body wastes discharged from a body wastes discharge hole or opening of a patient's body are received and stored, said pouch member being adapted to be detachably secured to or around the body wastes discharge hole or opening of the human body.

The body wastes receiving pouch appliance according to the present invention can be constituted in such a manner that a deodorant pouring inlet port is provided in the upper portion of the pouch member of the body wastes receiving pouch appliance, and a non-return valve formed of a double-layer film is provided in the pouch member so as to communicate with said deodorant pouring inlet port.

Further, the body wastes receiving pouch appliance according to the present invention can alternatively be constructed in such a manner that a deodorant pouring inlet port is provided in the upper portion of the pouch member of the body wastes receiving pouch appliance, and a non-return valve is sprovided as a valve mechanism in said deodorant pouring inlet port.

### [Brief description of the Drawings]

Fig. 1 shows an embodiment of the present invention, in which a) is a front view of said embodiment, b) is a longitudinal sectional side view of the same embodiment, and c) and d) are schematic diagrams explaining how this embodiment is used.

Fig. 2 shows a further embodiment of the present invention, in which a) is a front view of said further embodiment, b) is a longitudinal sectional side view of the same embodiment, and c) and d) are schematic diagrams explaining how the further embodiment is used.

### [Optimum Embodiments of the Invention]

The embodiments of the present invention will now be described referring to the drawings.

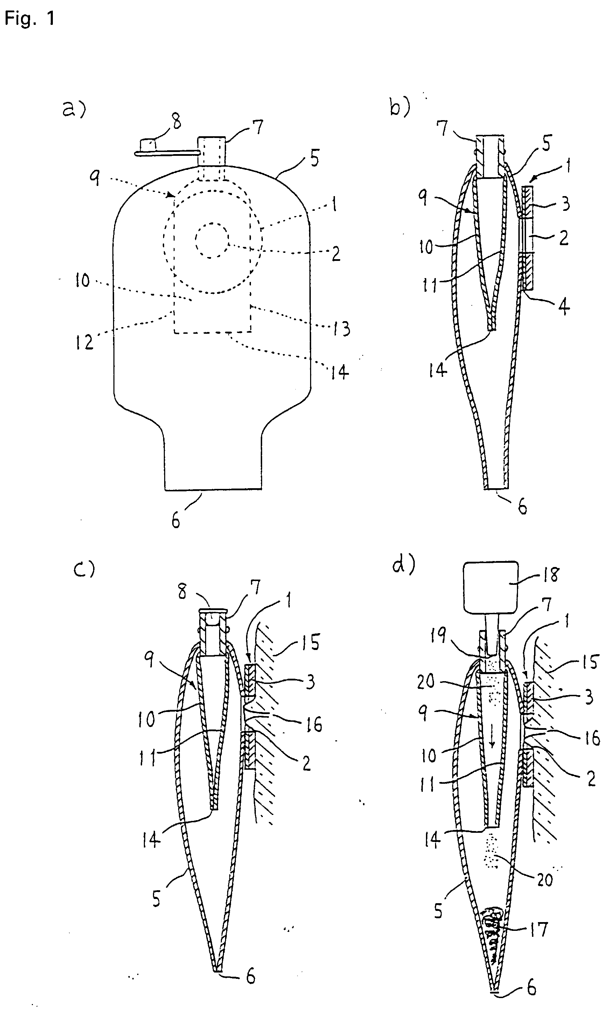

In Fig. 1, a) is a front view of an embodiment of the present invention, b) is a longitudinal sectional side view of the same embodiment, c) is a schematic diagram explaining how the body wastes receiving pouch appliance according to the embodiment is put on, and d) is a schematic diagram explaining the operation performed for pouring the deodorant into the pouch member. The reference numeral 1 denotes an adhesive plate which is, for instance, in a disk-like shape and has a central opening 2 corresponding to a body wastes discharge opening of a human body around which the body wastes receiving pouch appliance is to be secured, and, on the adhesive side of said adhesive plate 1, an adhesive layer 3 is provided, while on the surface thereof at the non-adhesive side - that is, the opposite side with reference to said adhesive side - thereof, a laminated film 4 is provided. Numeral 5 denotes a pouch member coupled to the peripheral area surrounding the opening 2 at the non-adhesive side of the adhesive plate 1. Said pouch member 5 functions as a container for receiving and storing the body wastes discharged through the opening 2 of the adhesive plate 1 from the body wastes discharge opening of the human body, and a lower end 6 of said pouch member 5 is open for allowing the removal of the body wastes from within the pouch member 5 but can be closed when used. The pouch emmber 5 is made of a plastics film which should desirably be water-insoluble and have excelllent gas barrier properties, flexibility and sealing properties; as said plastics film, the plastics films used as the materials of the pouch members of the conventional body wastes receiving pouch appliances can also be used; more specifically, the following can be pointed out: Single-layer films or composite films made of for instance polyvinylidene chloride, polyethylene, polyvinyl chloride and chlorinated polyethylene or the materials obtained by blending or copolymerizing vinyl acetate, polyacrylic acid or the like with the above-mentioned substances.

Numeral 7 denotes a deodorant pouring inlet port provided in the upper portion of the pouch member 5, which port is adapted in such a manner that the outlet of a deodorant container to be described later can be inserted into said pouring inlet port 7 to pour the deodorant into the pouch member 5. Numeral 8 denotes a lid belonging to said deodorant pouring inlet port 7. Said lid 8 is normally fitted into said pouring inlet port 7 to hermetically seal the pouch member 5 but can be removed when the deodorant is to be poured into the pouch emmber 5. The deodorant pouring inlet port 7 and the lid 8 can be made of the same material as that of the pouch member 5. The pouring inlet port 7 is formed in the shape of a tube with a wall thickness of 0.5 to 2 mm, but can also be made of a hard material, instead. Numeral 9 denotes a non-return valve coupled to the deodorant pouring inlet port 7. Said non-return valve 9 can be formed, for instance, in such a manner that two sheets 10 and 11 are put together, wherein side portions 12 and side portions 13 thereof are closed together, respectively, but lower end portions 14 thereof are made to form an open free end portion. The non-return valve 9 is formed by the use of a thin rubber film or a flexible plastics film but can alternatively be made of the same material as that of the pouch member 5. As for the length of said non-return valve 9, said valve 9 must be long enough to allow the lower end portions 14 of said two component sheets 10 and 11 to be naturally brought into close contact with each other.

Next, the way of using the body wastes receiving pouch appliance according to the present invention will be described. Referring to Fig. 1c), the body wastes receiving pouch appliance is fixed to or around a body wastes discharge opening 16 of a human body 15 by means of the adhesive layer 3 of the adhesive plate 1, wherein the lower end 6 of the body wastes receiving pouch appliance is closed up by fastening and the deodorant pouring inlet port 7 is plugged up with the lid 8. In this case, the non-return valve 9 is left to hang down, and the lower end portions 14 - including the portions in the vicinity of thereof - of the sheets 10 and 11 are in a state closely attached to each other, so that, even if some body wastes are present in the pouch member 5, they are not allowed to leak out through the non-return valve 9, and the odor of said body wastes is also prevented from leaking out. It is now to be assumed that some body wastes 17 are stored in the pouch member 5 as shown in Fig. 1d), and thus, it has become necessary to take out said body wastes for removal thereof or to replace the pouch emmber with a new one. Then, the lid 8 is drawn out from the deodorant pouring inlet port 7, an outlet 19 of a container 18 in which a deodorant 20 is contained is inserted into the deodorant pouring inlet port 7, and the deodorant is pushed out, whereby the lower end portions 14 of the two sheets 10 and 11 constituting the non-return valve 9 are pushed open, and thus, the deodortant 20 flows into the pouch member 5 to effect a deodorizing action, whereby the odor of the body wastes is almost completely removed. At this point of time, the lower end 6 of the pouch membe 5 is opened or the adhesive plate 1 is released from the body wastes discharge opening of the patient's body, whereby it is ensured that the unpleasant odor of the body wastes never spreads around.

As the deodorant, either the liquid type or the powder type can be used. As liquid type deodorants, the following ones can be pointed out by way of example: "Super Vanish" (trade name) manufactured by Smith & Nephew Inc., U.S.A., "M9" (trade name) manufactured by Sandack Inc., Denmark, "Charm Air" (trade name) manufactured by Eipack Corp., Japan, etc., while, as power type deodorants, the following ones can be pointed out: "Celler Filter" (trade name) manufactured by Nipro Corp., Japan, etc. The amount of the deodortant discharged from the container into the pouch member can be adjsuted depending on the amount of body wastes stored in the pouch member.

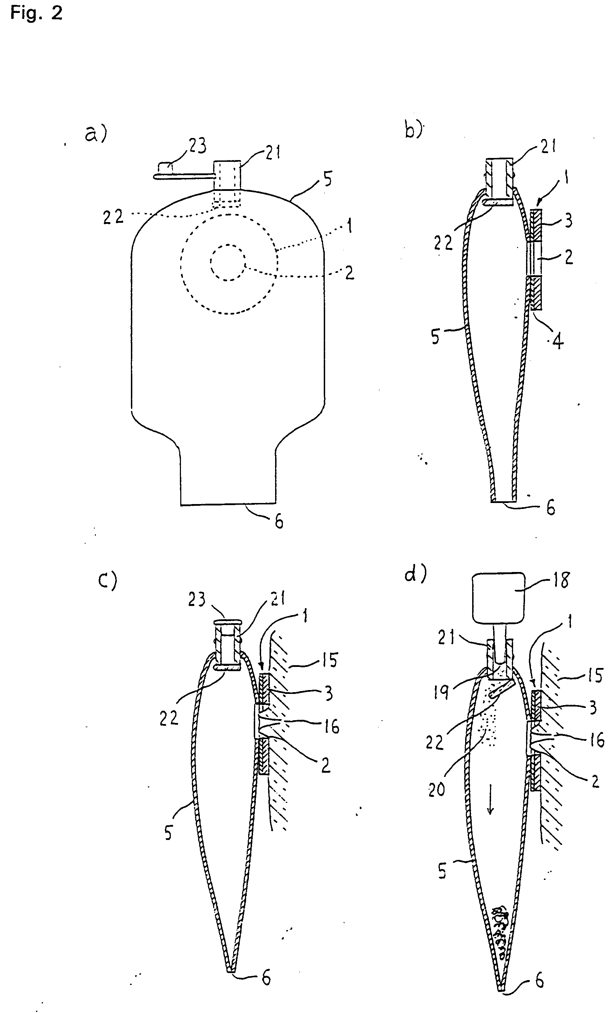

Fig. 2 shows a further embodiment of the present invention, in which a) is a front view of said further embodiment, b) is a longitudinal sectional side view thereof, c) is a schematic diagram explaining the state in which the body wastes receiving pouch appliance according to this embodiment is secured to a patient's body, and d) is a schematic diagam explaining how the body wastes receiving pouch appliance according to this embodiment is used, wherein the same portions as those shown in Fig. 1 are referenced by the same reference numerals. In this embodiment, in the upper portion of the pouch member 5, a deodorant pouring inlet port 21 provided, and this deodorant pouring inlet port 21 has a valve 22 at the lower end thereof. Said valve 22 has a reverse flow preventing function; that is, said valve 22 is adapted to be opened in the flowing-in direction in which fluid flows from the outside into the inside of the pouch member 5 but closed in the flowing-out direction in which fluid flows from the inside to the outside of the pouch member 5. Numeral 23 denotes a lid belonging to the deodorant pouring inlet port 21. Said lid 23 is normally fitted into the pouring inlet port 21 but taken off when the deodorant is to be poured in. The deodorant pouring inlet port 21, the valve 22 and the lid 23 can be made of the same material as that of the pouch member 5. Said pouring inlet port 21 is formed in the shape of a tube with a wall thickness of 0.5 to 2 mm, but can also be made of a different hard material, instead. The valve 22 can be formed in such a manner that it normally closely attaches to the valve seat due to the elasticity of the material thereof but is opened by the pressure of the deodorant poured in as will be described later or pushed down by the tip end of the deodorant container inserted into the pouring inlet port 21. As for the way of using this body wastes receiving pouch appliance, as shown in Fig. 2d), the outlet 19 of the container 18 containing a deodorant therein is inserted into the deodorant pouring inlet port 21, and the deodorant is pushed out, whereby the valve 22 is pushed open, and the deodorant is discharged into the pouch member 5. Then, by drawing out the container 18 from the pouring inlet port 21, the valve 22 is closed again. In the case of this embodiment, the same deodorant as that used in the foregoing embodiment can also be used.

According to the present invention, immediately before the body wastes are removed from within the pouch member or immediately before the pouch member is removed from the body wastes discharge opening of the patient's body, the deodorant is discharged into the pouch member through the pouring inlet port, whereby the deodorizing effect can be most effectively exhibited, so that the diffusion or spreading of the odor at the time of releasing the pouch member can be surely prevented, so that the body wastes receiving pouch appliance according to the present invention can be handled far more pleasantly and easily as compared with the conventional ones; and the deodorant can be prepared separately from the body wastes receiving pouch appliance, so that it can be ensured that the deodorant in an optimum amount corresponding to the amount of body wastes stored in the pouch member is poured into said pouch member.

## Claims

1. Body wastes receiving pouch appliance comprising a pouch member which is detachably secured to the body wastes discharge hole or opening of a human body, wherein body wastes can be received and stored in said pouch member characterized in that a deodorant pouring inlet port and a non-return valve are provided.

2. Body wastes receiving pouch appliance according to Claim 1, wherein the deodorant inlet port is provided in the upper portion of the pouch member, and the non-return valve is formed of a double-layer film in the pouch member so as to communicate with the deodorant pouring inlet port.

3. Body wastes receiving pouch appliance according to Claim 1, wherein the deodorant pouring inlet port is provided in the upper portion of the pouch member, and the non-return valve is built, as a valve mechanism, in said deodorant pouring inlet port.
